(19) Europäisches Patentamt
European Patent Office
Office européen des brevets

(11) Numéro de publication: **0 221 815**
**B1**

(12) **FASCICULE DE BREVET EUROPEEN**

(45) Date de publication du fascicule du brevet: **02.08.89**

(51) Int. Cl.⁴: **C07C 59/52**, C07C 59/64, C07C 51/377, C07D 333/24, C07D 317/60

(21) Numéro de dépôt: **86402336.1**

(22) Date de dépôt: **17.10.86**

(54) **Procédé pour la fabrication d'acides alkanoiques.**

(30) Priorité: **21.10.85 FR 8515572**

(43) Date de publication de la demande: **13.05.87 Bulletin 87/20**

(45) Mention de la délivrance du brevet: **02.08.89 Bulletin 89/31**

(84) Etats contractants désignés: **AT BE CH DE ES FR GB IT LI LU NL SE**

(56) Documents cités:
**FR-A- 2 415 109**
**FR-A- 2 470 127**

**L.F. FIESER ET M. FIESER: "Lehrbuch der organischen Chemie", édition 3, 1957, pages 770,771, Verlag Chemie GmbH, Weinheim, DE**

(73) Titulaire: **SOCIETE FRANCAISE HOECHST Société anonyme dite:, 3, avenue du Général de Gaulle, F-92800 Puteaux(FR)**

(72) Inventeur: Vallejos, Jean-Claude, 9 Rue Labat, **F-75018 Paris(FR)**
Inventeur: **Christidis, Yani, 53 Boulevard de la Villette, F-75019 Paris(FR)**

(74) Mandataire: **Rinuy, Santarelli, 14, avenue de la Grande Armée, F-75017 Paris(FR)**

**EP 0 221 815 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne un procédé de fabrication industrielle d'acides alkanoïques, plus particulièrement elle se rapporte à un procédé de fabrication d'acides acétiques substitués, ci-après désignés acides arylacétiques, de formule générale I :

```
Ar-CH-COOH        I
   |
   R
```

dans laquelle R représente un atome d'hydrogène ou un radical alkyle en $C_1$-$C_4$ et Ar représente un radical à caractère aromatique choisi parmi les radicaux suivants: thiényle-2, (méthoxy-2 naphtyle)-1, méthylènedioxy-3,4 phényle et les phényles substitués de formule générale II :

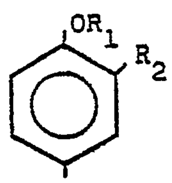

II

où $R_1$ représente un atome d'hydrogène ou un groupement alkyle en $C_1$-$C_4$ et $R_2$ représente un atome d'hydrogène, un atome d'halogène, un groupement alkyle, alcoxyle ou hydroxyle. Les acides arylacétiques de formule générale I sont largement décrits dans la littérature. Ce sont notamment des matières premières intéressantes pour la synthèse de substances pharmaceutiques actives.

Il est donc d'un grand intérêt de pouvoir les préparer d'une manière aussi rationnelle que possible.

On connait de très nombreuses méthodes pour préparer des acides acétiques substitués. Parmi celles-là, la réaction de Willgerodt-Kindler qui autorise la transformation d'une arylalkylcétone en acide arylalkylcarboxylique est l'une des plus connues. Toutefois, elle ne fournit généralement que des rendements modérés à médiocres et elle conduit à des produits secondaires soufrés responsables d'une pollution inacceptable aujourd'hui. Pour remédier à ces inconvénients, on s'est adressé à des méthodes plus elaborées necessitant l'emploi de plusieurs stades reactionnels tels que notamment a l'hydrogenolyse soit chimique, soit catalytique, d'acides arylglycoliques, d'acides o-acyle arylglycoliques ou d'acides aryl-2 halogeno-2 alkanoiques, issus dans certains cas de la condensation de l'acide glyoxylique sur le derive aromatique correspondant (demandes de brevet européens No 0.032.374 et 0.028.375, brevet européen No 0.003.825), l'hydrolyse d'un arylalkylacétonitrile obtenu par réaction de l'ion cyanure sur une benzylamine quaternisée convenablement substituée (demande de brevet européen No 0.062.440). Plus particulièrement, il est connu de réduire l'acide mandélique en acide phénylacétique par le melange iodure de potassium-phosphore rouge-acide orthophosphorique (L.F. Fieser et M. Fieser, Lehrbuch der Organischen Chemie, 3ème édition, 1957, page 771, Verlag Chemie GmbH, Weinheim, DE) et le thiophène-2 glycolate de sodium en acide thiophène-2 acétique au sein de l'acide acétique par le mélange soit acide iodhydrique à 57%-phosphore rouge soit acide chlorhydrique phosphore rouge-quantités cartalytiques d'acide iodhydrique à 57% (brevet français No 2.470.127). Ces methodes, dont certaines sont très récentes, exigent comme matière première de départ des produits rarement disponibles sur le marché, ce qui nécessite leur préparation préalable avec les inconvénients que cela comporte.

Or la Demanderesse a découvert avec étonnement qu'il était possible d'obtenir économiquement et avec de bons rendements et en un seul stade réalisé en phase homogène, des acides arylacétiques de formule générale I par réduction à chaud, en milieu acide, par de l'acide phosphoreux en présence de quantités catalytiques d'iode ou d'acide iodhydrique, d'un acide carboxylique alpha hydroxylé de formule générale III:

```
Ar-CR-COOH
   |
   OH            III
```

dans laquelle Ar et R ont la signification donnée précédemment.

Plus particulièrement, le procédé selon la présente invention consiste à réduire à une température supérieure à 70°C, en milieu acide, une mole d'un acide carboxylique alpha hydroxylé, de formule générale III, avec au moins un équivalent molaire d'acide phosphoreux en présence de 0,01 à 0,1 mole d'iode ou d'acide iodhydrique, éventuellement au sein d'un solvant organique compatible tel que notamment l'acide acétique dans laquelle Ar et R ont la signification donnée précédemment.

Plus particulièrement, le procédé selon la présente invention consiste à réduire à une température supérieure à 70°C, en milieu acide, une mole d'un acide carboxylique alpha hydroxylé, de formule générale III, avec au moins un équivalent molaire d'acide phosphoreux en présence de 0,01 à 0,1 mole d'iode ou d'acide iodhydrique, éventuellement au sein d'un solvant organique compatible tel que notamment l'acide acétique, puis à isoler l'acide cherché de formule générale I par des moyens connus en soi.

Avantageusement, le procédé de la présente invention consiste à réduire au reflux du milieu réactionnel une mole d'un acide carboxylique alpha hydroxylé de formule générale III avec une mole d'acide phosphoreux et 0,05 mole d'iode ou d'acide iodhydrique puis à isoler l'acide cherché par des moyens connus en soi.

L'acide iodhydrique est l'acide iodhydrique commercial en solution aqueuse à 57 % en poids.

L'acide carboxylique alpha hydroxylé est notamment l'acide parahydroxymandélique, l'acide méthylènedioxy-3,4 mandélique, l'acide hydroxy-4 méthoxy-3 mandélique, l'acide chloro-3-hydroxy-4 mandélique, l'acide thiophène-2-glycolique.

En fin de réaction, l'acide arylacétique cherché de formule générale I est isolé du milieu réactionnel par des moyens connus en soi. Généralement, après élimination des solvants réactionnels et neutralisation des acides minéraux présents par un sel d'acide faible tel que l'acétate de sodium, on reprend le milieu réactionnel avec de l'eau puis on élimine si nécessaire l'acide de départ non transformé par des moyens connus en soi puis on isole l'acide cherché soit par cristallisation, soit par tout autre moyen connu en soi.

Avec certains substrats, il est parfois avantageux d'isoler l'acide arylacétique cherché sous forme d'un de ses sels tels que notamment son sel de sodium, puis de déplacer ce sel par un acide minéral fort.

Le procédé de la présente invention permet d'obtenir, entre autres, les produits suivants :
- l'acide parahydroxyphénylacétique,
- l'acide hydroxy-4 méthoxy-3 phénylacétique,
- l'acide méthylènedioxy-3,4 phénylacétique.

D'autres caractéristiques de la présente invention apparaitront à la lecture de la description suivante et des exemples donnés à titre illustratif et nullement limitatif.

Exemple 1

Dans une solution chauffée au reflux contenant:
- 0,5 mole d'acide phosphoreux,
- 25 mmoles d'acide iodhydrique à 57 %
- 78 g d'acide acétique,

on introduit en 4 heures une solution chaude (40°C) constituée de :
- 0,5 mole d'acide parahydroxymandélique,
- 180 g d'acide acétique.

Le milieu réactionnel est ensuite maintenu 30 minutes au reflux, puis on le concentre sous vide après introduction de 8 g d'acétate de sodium anhydre. Le résidu cristallisé obtenu est ensuite chauffé au reflux pendant 90 minutes dans 350 g d'eau, puis après refroidissement à 40°C, il est traité avec 4 g de disulfite de sodium. Le milieu réactionnel est ensuite refroidi à 0°C, puis le précipité est filtré, lavé à l'eau glacée et enfin séché à 50°C sous vide à poids constant. On obtient ainsi 72,4 g d'acide parahydroxyphénylacétique pur soit un rendement de 95,3% de la théorie calculée par rapport à l'acide parahydroxymandélique mis en oeuvre.

Exemple 2

Une solution de 50 mmoles d'acide hydroxy-4 méthoxy-3 mandélique dans 18 g d'acide acétique est introduite en 75 minutes dans une solution de 50 mmoles d'acide phosphoreux, 2,5 mmoles d'acide iodhydrique à 57% et 8 g d'acide acétique maintenue à 95°C. A la fin de l'introduction, on poursuit le chauffage à 95°C pendant 45 minutes puis on introduit 0,4 g d'acétate de sodium avant de concentrer le milieu réactionnel sous vide. Le résidu cristallisé obtenu est dissous dans 20 g d'eau à chaud puis, si nécessaire, on décolore la solution obtenue par addition de disulfite de sodium et ensuite, on l'abandonne à la cristallisation à 0°C. On filtre le produit cristallisé, on le lave à l'eau puis on le séche à 50°C sous vide à poids constant. On isole ainsi 33,8 mmoles d'acide hydroxy-4 méthoxy-3 phénylacétique pur.

Exemple 3

En opérant selon l'exemple 2 au départ de l'acide méthylènedioxy-3,4 mandélique, on obtient avec un rendement de 73,4 % de la théorie l'acide méthylènedioxy-3,4 phénylacétique pur.

Exemple 4

Dans une solution de 0,5 mole d'acide phosphoreux, 6,25 mmoles d'iode dans 78 g d'acide acétique maintenue au reflux, on introduit en deux heures une solution de 0,5 mole d'acide parahydroxymandélique dans 180 g d'acide acétique préalablement chauffée à 40°C. A la fin de l'introduction, on maintient le milieu réactionnel une heure au reflux, puis on le concentre sous vide après addition de 2 g d'acétate de sodium anhydre. Le résidu cristallisé est ensuite dissous au reflux dans 250 g d'eau, puis la solution est traitée avec 4 g de disulfite de sodium avant d'être abandonnée à la cristallisation à 0°C. Le précipité cristallisé est ensuite filtré, lavé à l'eau et séché à 50°C sous vide à poids constant. On obtient ainsi 0,37 mole d'acide parahydroxyphénylacétique pur.

Il va de soi que la présente invention n'a été décrite qu'à titre purement explicatif et nullement limitatif et que toute modification utile pourra y être apportée sans sortir de son cadre.

## Revendications

1. Procédé de préparation d'acides acétiques substitués de formule générale I :

Ar-CHR-COOH I

dans laquelle R représente un atome d'hydrogène ou un radical alkyle en $C_1$-$C_4$ et Ar représente un radical à caractère aromatique choisi parmi les radicaux suivants: thiényle-2, méthoxy-2 naphtyle-1, méthylènedioxy-3,4 phényle, et les phényles substitués de formule générale II :

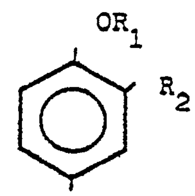

II

où $R_1$ représente un atome d'hydrogène ou un groupement alkyle en $C_1$-$C_4$ et $R_2$ représente un atome d'hydrogène, un atome d'halogène, un groupement alkyle, alcoxyle ou hydroxyle, procédé caractérisé par le fait que l'on réduit à chaud en milieu acide, par de l'acide phosphoreux en présence de quantités catalytiques d'iode ou d'acide iodhydrique, un acide carboxylique alpha hydroxylé de formule générale III :

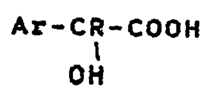

III

dans laquelle Ar et R ont la signification donnée précédemment.

2. Procédé selon la revendication 1, caractérisé par le fait que l'on réduit à une température supérieure à 70°C, en milieu acide et en phase homogène, une mole d'acide carboxylique alpha hydroxylé de formule générale III telle que définie à la revendication 1 par au moins une mole d'acide iodhydrique, éventuellement au sein d'acide acétique.

3. Application du procédé selon l'une quelconque des revendications 1 ou 2 à la fabrication de l'acide parahydroxyphénylacétique.

4. Application du procédé selon l'une quelconque des revendications 1 ou 2 à la fabrication de l'acide hydroxy-4 méthoxy-3 phénylacétique.

5. Application du procédé selon l'une quelconque des revendications 1 ou 2 à la fabrication de l'acide méthylènedioxy-3,4 phénylacétique.

**Patentansprüche**

1. Verfahren zur Herstellung von substituierten Essigsäuren der allgemeinen Formel (I)

Ar–CHR–COOH

worin R ein Wasserstoffatom oder einen $C_1$–$C_4$-Akylrest bedeutet und Ar einen Rest mit aromatischem Charakter ausgewählt aus folgenden Resten: 2-Thienyl, 2-Methoxy, 1-Naphthyl, 3, 4-Methylendioxy, Phenyl und substituierten Phenylen der allgemeinen Formel (II)

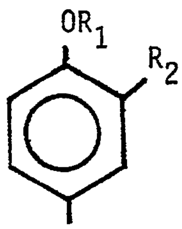

(II)

darstellt, worin $R_1$ ein Wasserstoffatom oder eine $C_1$–$C_4$–Alkylgruppe ist und $R_2$ ein Wasserstoffatom, ein Halogenatom, eine Alkyl-, Alkoxy- oder Hydroxylgruppe bedeutet, dadurch gekennzeichnet, daß man eine α-hydroxylierte Carbonsäure der allgemeinen Formel (III)

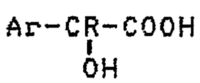

(III),

worin Ar und R die oben angegebene Bedeutung haben, unter Erhitzen in saurem Medium durch phosphorige Säure in Anwesenheit katalytischer Mengen Jod oder Jodwasserstoffsäure reduziert.

2. Verfahren nach Anspruch 1, dadurch gekennzeichnet, daß man bei einer Temperatur von höher als 70°C in saurem Medium und in homogener Phase ein Mol α–hydroxylierte Carbonsäure der allgemeinen Formel (III), wie in Anspruch 1 definiert, durch mindestens einem Mol Jodwasserstoffsäure, gegebenenfalls in Essigsäure, reduziert.

3. Anwendung des Verfahrens nach einem der Ansprüche 1 oder 2 bei der Herstellung von p-Hydroxyphenylessigsäure.

4. Anwendung des Verfahrens nach einem der Ansprüche 1 oder 2 bei der Herstellung von 4-Hydroxy-3-methoxy-phenylessigsäure.

5. Anwendung des Verfahrens nach einem der Ansprüche 1 oder 2 bei der Herstellung von 3,4-Methylendioxyphenylessigsäure.

**Claims**

1. Process für preparing substituted acetic acids of general formula I:

Ar-CHR-COOH

in which R represents an atom of hydrogen or a $C_1$–$C_4$ alkyl radical and Ar represents a radical of aromatic character chosen from the following radicals: 2-thienyl, 1-(2-methoxy-naphthyl), 3,4-methylenedioxy phenyl and substituted phenyls of the general formula II:

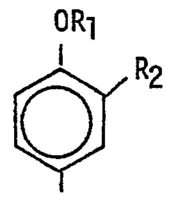

II

where $R_1$ represents an atom of hydrogen or a $C_1$–C4 alkyl group and $R_2$ represents a hydrogen atom, a halogen atom, or an alkyl, alkoxyl or hydroxyl group, said process being characterized in that it consists in the hot reduction, in an acid medium, by phosphorous acid in the presence of iodine or hydriodic acid, of an alpha hydroxylated carboxylic acid of the general formula III:

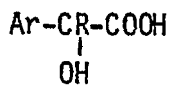

III

in which Ar and R have the same meaning as above.

2. Process according to Claim 1, characterized in that one mole of alpha hydroxylated carboxylic acid of general formula III such as that defined in Claim 1 is reduced at a temperature above 70°C, in an acid medium, in a homogenous phase, by at least one mole of hydrogen iodide, optionally in acetic acid.

3. Application of the process according to any one of Claims 1 or 2 to the manufacture of parahydroxyphenylacetic acid.

4. Application of the process according to any one of Claims 1 or 2 to the manufacture of 4-hydroxy-3-methoxy-phenylacetic acid.

5. Application of the process according to any one of Claims 1 or 2 to the manufacture of 3, 4-methylenedioxy-phenylacetic acid.